(19) Europäisches Patentamt
European Patent Office
Office européen des brevets

(11) EP 1 510 137 A1

(12) EUROPEAN PATENT APPLICATION

(43) Date of publication:
**02.03.2005 Bulletin 2005/09**

(51) Int Cl.[7]: **A23L 1/00**, A61K 35/78, A61P 39/06

(21) Application number: **04077281.6**

(22) Date of filing: **12.08.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **14.08.2003 US 604749**

(71) Applicant: **Alberta Research Council, Inc.**
**Edmonton, Alberta T6N 1E4 (CA)**

(72) Inventors:
- **Gandhi, Neena**
  **Edmonton, Alberta T6N 1E4 (CA)**
- **Wettasinghe, Mahinda**
  **Edmonton, Alberta T6N 1E4 (CA)**

(74) Representative: **Wilson Gunn Skerrett**
**Charles House**
**148/9 Great Charles Street**
**Birmingham B3 3HT (GB)**

(54) **Pyruvate enriched onion extract**

(57) Extracts of plants of the genus *Allium,* such as onion bulbs (*Allium cepa*), contain pyruvic acid and possess the ability to inhibit generation of organic free radicals, quench pre-formed free radicals, and to induce the activity of antioxidant enzymes such as quinone reductase, thus limiting damage due to aging such as cataracts.

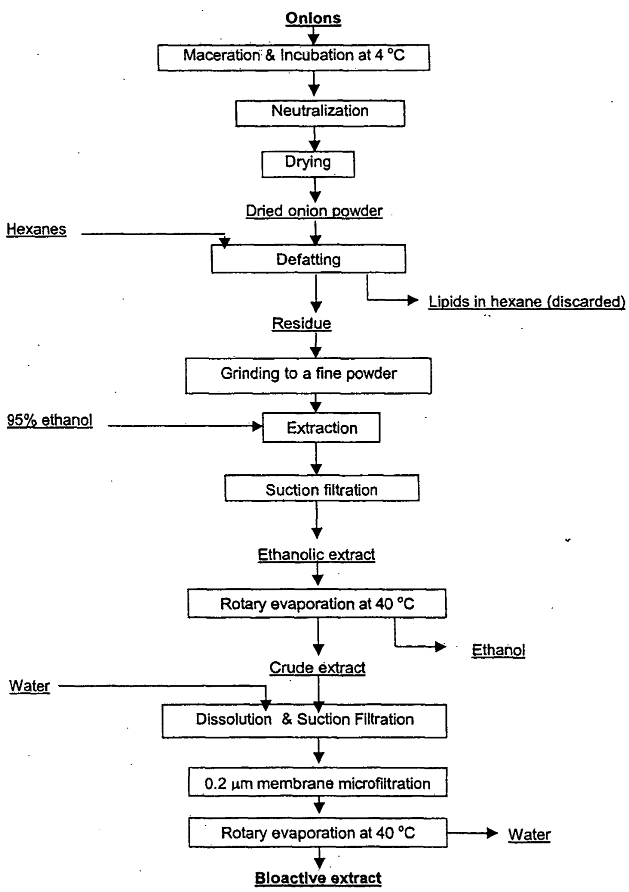

Figure I: Onion bulb extraction scheme

EP 1 510 137 A1

## Description

## FIELD OF THE INVENTION

The present invention relates to an antioxidant extract derived from onion bulbs as well as a process for preparing the antioxidant extract and uses for the antioxidant extract.

## BACKGROUND OF THE INVENTION

Considerable attention has been focused in recent years on the negative health effects of free radicals. These unstable, reactive substances can cause severe damage to the cell structure. Generation of free radical species beyond the antioxidant capacity of a biological system gives rise to oxidative stress. There is evidence that oxidative stress plays a role in heart diseases, neurodegenerative disorders, cancer and the aging process. Therefore, lowering oxidative stress in bodily tissues by maintaining a healthy level of antioxidants can slow down or prevent the onset of these diseases. In addition, antioxidants are used as preservatives during food manufacture to minimize peroxidation of food lipids.

Antioxidants can protect food and membrane lipids by several means, but most important is their ability to neutralize free radicals that propagate self-catalyzed chain-reactions. Neutralization occurs when an antioxidant donates either an electron or a hydrogen atom to the free radical. In this process, antioxidant itself becomes a free radical, but the free electron is stabilized by resonance or condensation. Both flavonoids and keto-acids have these functionalities.

Plant materials contain many different antioxidant compounds. Plant-derived antioxidants, such as tocopherols, ascorbic acid and flavonoids are important dietary antioxidants that may protect humans against free radical-mediated diseases. Onions are a rich source of keto-acid antioxidants such as pyruvic acid and flavonoids such as quercetin and kaempferol.

A variety of plant-derived antioxidants are commercially available. These include extracts of garlic, *Gingko biloba*, berries, rosemary, sage, thyme, soybean, tea, oregano and the like. In addition, various types of onion extracts are available commercially, but these extracts are intended for use as flavoring agents and no attempt has been made to enrich their antioxidant, primarily pyruvate, content or apply them for this content.

Considerable research has been carried out on the antioxidant capacity of pyruvate. At one time, it appeared that natural antioxidants such as vitamins A, C, E and various minerals were thought to hold great promise in combating free radicals, but now it appears they are only marginally effective. Pyruvate, however, has been proven in studies on mammals to be both a free radical scavenger and inhibitor. This is significant because there are various substances that supposedly act as free radical scavengers in the body, but pyruvate is unique in that it also acts as an inhibitor to the production of these reactive species.

A major function of pyruvic acid is energy release as it is instantly incorporated into the catabolic pathways for energy such as the Krebs' cycle. In fact, pyruvate is one of the intermediate products of glucose metabolism for energy, and is consequently a more instant source of energy than even glucose. Besides this obvious importance of pyruvate, its supplementation is reported to confer health benefits such as improved eye health, enhanced endurance, weight loss, inhibition of ischemic heart and intestinal injury, decrease in osteoporosis, lowering of blood glucose levels in diabetes and inhibition of cancerous tumors.

Pyruvic acid is a three-carbon $\alpha$-keto-acid with antioxidative activity, which has been shown to be active in eye health, especially in the prevention and treatment of age-related disorders such as cataract and glaucoma. It is believed to help in scavenging of free radicals, prevention of lipid peroxidation in the retina, and prevention of glycation of the eye lens.

A cataract is the clouding of the lens of the eye, which reduces the amount of incoming light and results in deteriorating vision. Many factors influence vision and cataract development including age, sunlight and nutrition. The aging lens suffers metabolic changes that may predispose it to cataract development. Some of this occurs due to low supply of oxygen and nutrients, which leave the eye vulnerable for free radical damage. According to a 1983 report from the National Academy of Science, cataracts are initiated by free-radical hydrogen peroxide found in the aqueous humor. Free radicals such as hydrogen peroxide oxidize glutathione and destroy the energy-producing system of the eye and allow leakage of sodium into the lens. Water follows the sodium, and the edema phase of the cataract begins. Then, body heat oxidizes (cooks) lens protein in the eye, and it becomes opaque and insoluble (similar to egg protein).

Free radicals reside in the aqueous fluid and bathe the lens of the eye, destroying enzymes that produce energy and maintain cellular metabolism. Free radicals also break down fatty molecules in membranes and lens fibers, generating more free radicals and creating a cross-linking (denaturing or breakdown) of the laminated-like structural proteins inside the lens capsule. The lens capsule has the ability to swell or dehydrate. In doing so the increase and/or decrease in pressure can cause breaks in the lens fiber membranes, resulting in microscopic spaces in the eye in

which water and debris can reside. Glycation (glycosylation) of proteins has been shown to play a prominent role in the development of diabetic cataract formation and retinopathy. The glycosylation process also occurs as a result of general aging. This mechanism is also responsible for glaucoma, a major cause of age-related vision loss, which is also triggered off by oxidative stress.

The antioxidant activity of pyruvate and its effectiveness in cataract prevention and alleviation has been demonstrated through several animal studies and laboratory research

Pyruvate is commercially available in capsule and powder form, as well as in bars and drinks. Methods of production, which require technical expertise and sophisticated equipment, include biofermentation and biochemical synthesis. The stability and bioavailability of pyruvate in the end-product are also crucial and are important factors to consider. Pure pyruvic acid is highly unstable and strongly acidic. Pyruvic acid in salt form is more stable and neutral.

Bioavailability of pyruvic acid is low in the free acid form. Purified salts of pyruvic acid are also not physiologically suitable. Amino-compounds containing pyruvate such as pyruvylglycine lead to excessive nitrogen loads. Also, flooding plasma with glycine may interfere with the transport of some amino acids across the blood-brain barrier. Accordingly, these pyruvate compounds are less suited to treating an organ in vivo, and it is recognized that a need exists to provide a pyruvate delivery compound that is more physiologically acceptable.

Therefore, there is a need in the art for compositions comprising stable, bioavailable, physiologically acceptable and economic sources of pyruvate.

## SUMMARY OF THE INVENTION

In the present invention, pyruvate content of a plant extract has been enhanced but no attempt has been made for total purification so that it can be present in its natural milieu where it may be much more bioavailable and stable. Also, the presence of other antioxidants such as the flavonoids, quercetin and kaempferol, will result in a reinforcing of the antioxidant activity. This process of enhancing pyruvate content in plant extracts is novel and these extracts can be used to deliver pyruvates inexpensively to the consumers, as either nutraceutical or functional food ingredients, or both.

Therefore, in one aspect, the invention may comprise a method of producing an antioxidant composition, comprising the steps of:

(a) macerating tissue from plants of the genus *Allium* and incubating the tissue at a temperature less than about 30° C;

(b) neutralizing the macerated tissue with an alkali metal hydroxide or an alkaline earth hydroxide;

(c) drying the tissue;

(d) grinding the tissue;

(e) extracting the tissue with a solvent; and

(f) evaporating the solvent to recover a solid residue comprising pyruvate.

Preferably, the plant is selected from the group consisting of *Allium cepa* and *Allium sativum*. The tissue may be defatted after drying using a suitable solvent such as hexane.

In another aspect, the invention may comprise a pyruvate-enriched plant extract as prepared by a method described or claimed herein.

In another aspect, the invention may comprise a plant extract derived from tissue from plants of the genus *Allium* enriched in pyruvic acid by at least 100%.

## BRIEF DESCRIPTION OF THE DRAWINGS

The invention will now be described by way of an exemplary embodiment with reference to the accompanying simplified, diagrammatic, not-to-scale drawings. In the drawings, Figure 1 is a schematic representation of one embodiment of the present invention. Figure 2 is a standard curve for known amounts of pyruvate in a sample.

## DETAILED DESCRIPTION OF THE INVENTION

The present invention provides for a method of enhancing antioxidant levels in an extract derived from plants

of the genus *Allium.* When describing the present invention, all terms not defined herein have their common art-recognized meanings. As used herein, "pyruvic acid" and "pyruvate" are used interchangeably and mean a three-carbon organic acid having the following structure:

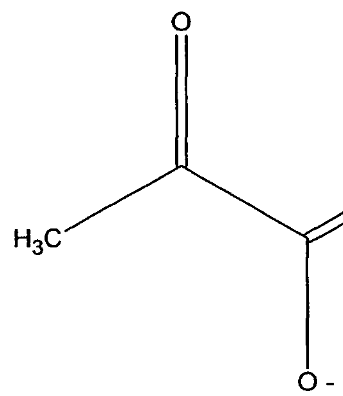

Plant of the genus *Allium* include various varieties of onions, *Allium cepa*, and garlic, *Allium sativa*. Onions are reported to have high pyruvate levels and are a preferred source of antioxidants. Garlic bulbs are also known to have relatively high pyruvate levels and are also rich in antioxidants. In intact onion bulb tissue, low levels of pyruvic acid are naturally present as it is an intermediate of the glycolytic pathway. The major pyruvic acid generation in onion tissue however, occurs enzymatically, in response to tissue damage (e.g. by maceration) through the activity of alliinase. The amount of pyruvic acid so generated is directly dependent upon the extent of enzyme activity and can therefore be maximized by providing the optimal conditions for these enzymes. Concentration techniques may then be used to enhance the yield.

Figure 1 shows the scheme for extraction of antioxidants from an *Allium* plant. Peeled onion bulbs are macerated and incubated. In a preferred embodiment, the macerated tissue is incubated at ambient temperature or less for at least about 5 minutes. In one embodiment, the tissue is incubated at 4° C for 30 minutes. During incubation, pyruvate is generated by enzymatic activity. After incubation, macerated onions are neutralized with an alkali metal (Group 1) or alkaline earth (Group 2) hydroxide, dried, defatted, and ground. The dried onion powder is then subjected to extraction with 95% ethanol at a temperature not exceeding about 60 °C for up to about 2 hours. The ethanolic extracts are rotary evaporated and the residue suspended in distilled water. This "crude extract" is suction-filtered and further subjected to membrane microfiltration.

The extraction steps yield a concentrated extract that is water-soluble, quenches the formation of ABTS (2-2'-azino-bis(3-ethylbenzathiazoline-6-sulfonic acid) free radicals, reduces pre-formed ABTS radicals and has oxygen radical absorbance capacity. The extract also possessed antioxidant activity which was was detected *in vitro* via quinone reductase induction in murine hepatoma cells.

Fresh onions are known to have pyruvate content in the range of 0.25 - 0.50 g per kg of onion. After maceration and incubation, the pyruvate content rises to at least about 0.70 g per kg. Therefore, enrichment of pyruvate in the extract over that in fresh onions is at least about 100%, preferably about 140% and more preferably may be as high as 250% or higher. The extract is characterized by pyruvate content in the range of about 1-7 % by weight. The flavonoids (0.2-0.3%) present include quercetin and kaempferol. The other known constituents of the extract include 10.5% proteins and 53% sugars.

As used herein, "enriched in pyruvic acid" means that the extract contains more pyruvic acid than the fresh onion tissue had prior to being processed. For example, if 1 kg of fresh onions containing 0.25 g of pyruvic acid results in an extract containing 0.50 g of pyruvic acid, then the extract is enriched by 100%.

Tests for stability determination confirmed that pyruvates in this form are more stable than pyruvic acid. Results showed that pyruvate loss, with or without added hydrogen peroxide, is 3% as compared to 11% and 26% loss observed for pyruvic acid with and without added hydrogen peroxide, respectively.

The ability of the extract to inhibit the formation of free radicals is described in terms of its ability to reduce ABTS radicals. The extract has a 34% inhibitory effect at an assay concentration of 454 µg/mL. The ability of the extract to quench pre-formed ABTS radicals is 41% at a similar assay concentration. 35% of oxygen radicals generated in an assay system could be neutralized by the bioactive extract.

Induction of the antioxidant enzyme, quinone reductase, a representative biomarker of cellular defense system, in mice hepatocytes was determined *in vitro.* Bioactive extracts at a dose of 509 µg per 10000 cells resulted in a

more than 6-fold increase in the enzyme activity compared to the control devoid of antioxidants. At this dose, the cytotoxicity of the extracts was within the acceptable limits (cell survival rate of ≥ 50%).

Thus, this bioactive extract could be applied towards anti-aging disorders as well as other ailments where the basic disruptive mechanism arises due to oxidation processes. It can be formulated in capsule forms as a dietary supplement or as a component of functional foods such as mayonnaise, bars or other foods.

The invention is further illustrated by the following examples, which are intended to be exemplary and not limiting of the claimed invention.

## EXAMPLE I: PREPARATION OF THE EXTRACT

### *(a) Enhancement of pyruvate production in situ*

Onions (1 kg) were refrigerated overnight, peeled, cut in half and crushed in a food processor. Crushed onions were incubated in an ice bath (4 °C) and at 25 °C for different durations. Sodium hydroxide was added to neutralize the slurry, mixed well and dried at 20 °C for 48 h. Dried onion was then defatted using hexanes and any residual hexane was evaporated. Defatted onion was then ground to a fine powder and subjected to extraction with 95% (v/v) ethanol at 50 °C for 2h. Liquid extracts were filtered under suction. The resultant ethanolic filtrate was rotary evaporated and the pyruvate concentration of the residue was determined.

Table 1 shows the pyruvate yield of residues obtained under different incubation conditions. About 2 to 3-fold enhancement in pyruvate yields could be obtained by incubation at 4 °C for 30 min.

Table 1:

| **Pyruvate yield as a function of incubation conditions** | | |
|---|---|---|
| Incubation time (min) | Pyruvate yield (mg) | |
| | 4 °C | 25 °C |
| 5 | 222 | 160 |
| 15 | 314 | 190 |
| 30 | 369 | 182 |
| 45 | 354 | ND |
| 60 | 374 | ND |

### (b) *Effect of extraction solvent and time*

Defatted onion was produced as in (a) above, ground to a fine powder and subjected to extraction with either 100 or 95% (v/v) ethanol at 50 °C for varying time periods. Liquid extracts were filtered through under suction. Ethanolic filtrate was rotary evaporated and the extract concentrated.

Table 2 shows the pyruvate yield of residues obtained using different extraction conditions.

Table 2:

| **Effect of extraction condition** | | | |
|---|---|---|---|
| Solvent | Extraction time (min) | Pyruvate (mg) | Percent of initial pyruvate extracted |
| 100% EtOH | 30 | 136.9 | 23 |
| 95% EtOH | 30 | 386.4 | 67 |
| 95% EtOH | 60 | 486.4 | 84 |
| 95% EtOH | 120 | 579.1 | 100 |

### *(c) Effect of neutralization agent*

Ethanolic filtrate was produced as in (a) above from Gouw Quality white onions using 1.005 N sodium hydroxide and 1.0 N calcium hydroxide for neutralization of separate batches. This was subj ected to rotary evaporation at 40 °C to remove ethanol, followed by micro filtration of the diluted residue under suction with 0.2 μm Nalgene cellulose

acetate membrane unit. The resulting permeate was again subjected to rotary evaporation at 50 °C to remove excess water. The residue from this is referred to herein as the "onion extract".

Results of neutralization with different agents are shown in Table 3. While the pyruvate yield was the same, the amount of extract obtained was less when sodium hydroxide was used, albeit with a higher pyruvate concentration as compared to when calcium hydroxide was employed.

Table 3:

| Effect of neutralization agent | | |
|---|---|---|
| | NaOH | $Ca(OH)_2$ |
| Pyruvate in fresh onion (g/kg) | 0.273 | 0.273 |
| Pyruvate in onion extract (g/kg onions) | 0.719 | 0.709 |
| Pyruvate conc in onion extract (wt%) | 1.83 | 1.38 |
| Amount of onion extract (g/kg onions) | 13.067 | 16.089 |
| Enrichment of pyruvate (%) | 264.2 | 260.4 |

**EXAMPLE II: CHARACTERIZATION OF THE EXTRACT**

***(a) Total pyruvate content***

Solutions were prepared by dissolving onion extracts in water. A series of aqueous standard solutions containing pyruvic acid at 0,0.026,0.052,0.104,0.207,0.414,0.829 and 1.66 mg/mL were used to determine the standard curve.

0.5 mL from each solution was transferred separately into 50 mL centrifuge tubes and allowed to stand for 10 min. A 1.5 mL aliquot of aqueous trichloroacetic acid (TCA, 5%, w/v) was added into each tube, vortexed and to this, 18 mL of distilled water was further added. Tubes were vortexed and a 1 mL aliquot transferred into 10 mL screw-capped test tubes. 1 mL of 0.0125% 2,4-dinitrophenylhydrazine (prepared in 2N hydrochloric acid) was then added into each tube, along with 1 mL of distilled water and vortexed. Tubes were then heated for 10 min at 37 °C. 5 mL of 0.6N sodium hydroxide was added into each tube, vortexed and the absorbance of the resulting brick-red complex was measured at 420 nm.

Absorbance values of samples were compared against standard values and concentration of samples calculated. 2-3 fold yields of pyruvate were obtained in the final extract as compared to the initial onions through the optimized processing scheme. A typical standard curve is shown in Figure 2.

***(b) Determination of flavonoids***

Fresh onion juice, and the pyruvate-enriched extract were subjected to acid hydrolysis in order to facilitate the liberation of bound flavonoids from flavonoid glycosides.

Extract (1.5 mL) in a screw-capped vial was mixed with 0.5 mL of 6N hydrochloric acid, heated at 95 °C for 30 min and 1 mL of distilled water was added. Ethyl acetate (1 mL) was added into the vial, vortexed and the organic layer separated. This was repeated twice more. Pooled ethyl acetate layer was dried under a stream of nitrogen, residue dissolved in 3 mL of 50% (v/v) acetonitrile, 0.2 μm cartridge filtered and submitted to HPLC. Quercetin and kaempferol in the extract were quantified using standard curves prepared for authentic quercetin and kaempferol.

Quercetin content in fresh onion juice and microfiltered extract was 0.05 and 0.20 (%w/w), respectively, while content of kaempferol was 0.003 and 0.02 (%w/w, respectively.

**EXAMPLE III: ANTIOXIDANT ACTIVITY**

***(a) Inhibition (%) of ABTS radical generation***

All reagents used in this assay were prepared in phosphate buffered saline (PBS, 10 mM, pH 7.4). Sample solutions were prepared in PBS.

100 μL of 2.5 mM ABTS (2-2'-azino-bis(3-ethylbenzathiazoline-6-sulfonic acid), 180 μL of 50 μM metmyoglobin, 790 μL PBS and 10 μL of onion extract (final assay concentration of 454 μg/mL) were mixed in a disposable cuvette. Reaction was triggered by the addition of 120 μL of 10 mM hydrogen peroxide. Absorbance data of the reaction mixture

were recorded after 10 min using a spectrophotometer set at 734 nm. Quercetin was used as a reference antioxidant.

Inhibition (%) of ABTS radical generation was calculated using the following equation:

$$\% \text{ inhibition} = [(Abs_{control} - Abs_{sample})/Abs_{control}]*100$$

Table IV shows the typical data obtained for standard quercetin and the onion extract.

Table 4:

| **Inhibition (%) of ABTS Radical Generation** | | | |
|---|---|---|---|
| Component concentration | % inhibition | | |
| | 5 mg/mL | 10 mg/mL | 20 mg/mL |
| PBS (Control) | 0 | 0 | 0 |
| Quercetin* | 23 | 23 | 23 |
| Onion extract | 16 | 18 | 34 |

* Concentration was 0.005 mg/mL

***(b) Total reducing power***

ABTS radical was generated by mixing 5 mL of an aqueous ABTS solution (7 mM) and 88 μL of a potassium persulfate solution (140 mM), followed by the standing in the dark for 6 h. This stock solution (1.1 mL) was diluted to 90 mL with PBS (pH 7.4) and the absorbance adjusted to 0.83. Test solution (10 μL, final assay concentration of 454 μg/mL) and PBS (pH 7.4, 190 μL) were transferred into a disposable cuvette and the assay was started by addition of 1 mL of the pre-formed ABTS radical. Absorbance at 734 nm was measured after 10 min. Quercetin was used as a reference antioxidant. Total reducing power (TRP, %) was calculated using the following equations:

$$TRP_{sample + PBS} (\%) = [(Abs_{0\ min} - Abs_{10\ min})/ Abs_{0\ min}]*100$$

$$TRP_{sample} = (TRP_{sample + PBS}) - TRP_{control}$$

Typical results are shown in Table 5.

Table 5:

| **Total reducing power** | | | |
|---|---|---|---|
| | % TRP | | |
| Component concentration | 5 mg/mL | 10 mg/mL | 20 mg/mL |
| PBS (Control) | 0 | 0 | 0 |
| Quercetin* | 30 | 30 | 30 |
| Onion extract | 13 | 20 | 41 |

* Concentration was 0.005 mg/mL

***(c) Oxygen radical absorbance capacity (ORAC)***

A solution of 2,2'-azobis-(2-amidinopropane) dihydrochloride (AAPH, 0.5 M) was prepared in degassed distilled water and a β-carotene solution was prepared by centrifuging 10 mg of β-carotene in 10 mL of acetone followed by a one-fold dilution of the supernatant with the same solvent. β-carotene (60 μL) and 0.6% (w/v) phosphate buffered Tween 20 containing 0.3% (w/v) of linoleic acid (120 μL) were mixed with 935 μL of PBS in a quartz cuvette and incubated for 2 min at 50 °C. 10 μL of sample solution (final assay concentration of 454 μg/mL) and AAPH (25 μL) were added into the cuvette and the reaction was monitored for 10 min using a spectrophotometer set at 452 nm. Quercetin was used as a reference antioxidant. Cuvette was maintained at 50 °C all times except when the measurement is being taken. Percentage β-carotene retention was calculated using the following equation:

$$\% \ \beta\text{-carotene retention} = 100 - [(Abs_{0\ min} - Abs_{5\ min}) *100/ Abs_{0\ min}]$$

Typical results are shown in Table 6.

Table 6:

| ORAC in terms of β-Carotene retention | | | |
|---|---|---|---|
| | % TRP | | |
| Component concentration | 5 mg/mL | 10 mg/mL | 20 mg/mL |
| PBS (Control) | 28 | 28 | 28 |
| Quercetin* | 55 | 55 | 55 |
| Onion extract | 25 | 35 | 38 |

* Concentration was 0.005 mg/mL

**EXAMPLE IV: BIOACTIVITY AND TOXICITY IN MICE HEPATOCYTES**

This assay used Hepa 1c1c7 murine hepatoma cells cultured in two 96-well microtitre plates. One plate was used for the quinone reductase (QR) assay while the other was for the total protein measurement. Each plate containing 10,000 cells in Minimum Essential Medium (MEM)-well was incubated for 24 h, emptied and then 200 μL of serially diluted onion extract (final concentration of 0-1108 μg/well) in MEM were added into the wells. In each plate, there were two lanes of wells devoted to a no-cell blank and a cell control of test materials. Wells in these two lanes contained MEM in place of test materials. After incubating for 48 h, the wells of one plate were emptied and the cells were lysed using 50 μL of 0.08% (w/v) aqueous digitonin solution. The plate was then incubated for 20 min in a shaker incubator at 37 °C and removed from the incubator. A 150 μL aliquot of an aqueous assay reagent containing fetal bovine serum (0.066%; w/v), Tris-Cl (2.5%, v/v), Tween 20 (0.67%, v/v), flavin-adenine dinucleotide (FAD, 0.67%, v/v), glucose-6-phosphate (0.1 %w/v), nicotinamide adenosine diphosphate (NADP; 0.002 %w/v), glucose-6-phosphate dehydrogenase (0.0007 %w/v), 3-(4,5-dimethylthiazol-2-yl)-2,5 diphenyltetrazolium bromide (MTT, 0.03 %w/v), menadione (0.0008 %w/v) and acetonitrile (0.1 %v/v; used to prepare menadione solution) was added into each well. The absorbance of the reduced tetrazolium dye was measured after a 5-min period using an optical microtiter plate scanner set at 490 nm.

The second plate was emptied, 200 μL of crystal violet solution transferred into each well. After 10 min, the plate was rinsed under cold running water to remove excess stain. A 200 μL aliquot of 0.5 % (w/v) of sodium dodecyl sulfate (SDS) solution (prepared in 50% aqueous ethanol) was added into each well and the plate was incubated for 1 h in a shaker incubator set at 37 °C. Plate was removed and the absorbance of the crystal violet was measured at 562 nm. The degree of staining as reflected by the absorbance values of crystal violet was used as a measure of cell density.

For a given test material, the QR specific activity as induced by test compounds was calculated using both the absorbance value at 5 min in the QR assay and the absorbance value of crystal violet assay. Ratio of QR specific activity was the ratio between treated and control.

Typical results are shown in Table 7. Onion extract was considered safe at a dosage level of 509 μg and it became toxic at the 1108 μg dose. It induced the expression of QR by several folds in a concentration-dependant manner. LD50 was estimated to be 2.545 mg/mL, corresponding to pyruvate and flavonoid concentrations of 0.819 mg/mL and 0.0056 mg/mL, respectively.

Table 7:

| Cell assay data* | | |
|---|---|---|
| Dose (μg) | Ratio of QR specific activity (treated/control) | Ratio cell density (treated/control) |
| 0 | | |
| 2 | 1.00 | 1.00 |
| 4 | 1.07 | 0.97 |
| 8 | 1.12 | 0.97 |

Table 7: (continued)

| Cell assay data* | | |
|---|---|---|
| Dose (μg) | Ratio of QR specific activity (treated/control) | Ratio cell density (treated/control) |
| 16 | 1.17 | 0.96 |
| 32 | 1.22 | 0.98 |
| 64 | 1.52 | 0.92 |
| 127 | 1.93 | 0.91 |
| 254 | 2.45 | 0.90 |
| 509 | 3.95 | 0.82 |
| 1108 | 6.60 | 0.49 |
| | toxic | 0.19 |
| • mean value of 8 replicates, error within 5% | | |

As will be apparent to those skilled in the art, various modifications, adaptations and variations of the foregoing specific disclosure can be made without departing from the scope of the invention claimed herein. The various features and elements of the described invention may be combined in a manner different from the combinations described or claimed herein, without departing from the scope of the invention.

**References:**

The following references are incorporated herein by reference, as if reproduced in their entirety.

Bacon JR, Moates GK, Ng A, Rhodes JC, Smith AC, Waldron KW. 1999. Quantitative analysis of flavour precursors and pyruvate levels in different tissues and cultivars of onion (*Allium cepa*). *Food Chem.* 64: 257-261.

Bilyk A, Cooper PL, Sapers GM. 1984. Varietal differences in distribution of quercetin and kaempferol in onion (*Allium cepa* L.) tissue. *J. Agric. Food Chem*. 32: 274-276.

Haldeman DJ, Macneil JH, Yared DM. 1987. Antioxidant activity of onion and garlic juices in stored cooked ground lamb. *J. Food Protect.* 50: 411-413.

Horbowicz M, Kotlinzka T. Level of flavonols in wild and cultivated *Allium* species. *Acta Hort*. 517: 375-380.

Karastogiannidou C. 1999. Effects of onion quercetin on oxidative stability of cook-chill chicken in vacuum containers. *J. Food Sci.* 64: 978-981.

Macone A, Nardini M, Antonucci A, Maggio A, Matarese RM. 2002. Identification of aminoethylcysteine ketimine decarboxylated dimmer, a natural antioxidant, in dietary vegetables. *J. Agric. Food Chem.* 50: 2169-2172.

Nuutila AM, Puupponen-Pimia R, Aarni M, Oksman-Caldentey K-M. 2003. Comparison of antioxidant activities of onion and garlic extracts by inhibition of lipid peroxidation and radical scavenging activity. *Food Chem.,* 81: 485-493.

Ou B, Huang D, Hampsch-Woodill M, Flanagan JA, Deemer EK. 2002. Analysis of antioxidant activities of common vegetables employing oxygen radical absorbance capacity (ORAC) and ferric reducing antioxidant power (FRAP) assays: a comparative study. *J. Agric. Food Chem.* 50: 3122-3128.

Paganga G, Miller N, Rice-Evans, CA. The polyphenolic content of fruit and vegetables and their antioxidant activities. What does a serving constitute? *Free Rad. Res.* 30: 153-162.

Price KR, Rhodes MJC. 1997. Analysis of the major flavonol glycosides present in four varieties of onion (*Allium cepa*) and changes in composition resulting from autolysis. *J. Sci. Food Agric.* 74: 331-339.

Proteggente AR, Pannala AS, Paganga G, van Buren L, Wagner E, Wiseman S, van de Put F, Dacombe C, Rice-Evans CA. 2002. The antioxidant activity of regularly consumed fruit and vegetables reflects their phenolic and vitamin C composition. *Free Rad. Res.* 36: 217-233.

Schwimmer S, Weston WJ. 1961. Enzymatic development of pyruvic acid in onion as a measure of pungency. *J. Agric. Food Chem*. 9: 301-304.

Shobana S, Naidu KA. 2000. Antioxidant activity of selected Indian species. *Prostaglandins Leukot. Essent. Fatty Acids* 62: 107-110.

Wall MM, Corgan JN. 1992. Relationship between pyruvate analysis and flavour perception for onion pungency determination. *Hort. Sci.* 27: 1029-1030.

Yoo, K.S., Pike, L.M. Determination of background pyruvic acid concentrations in onions, Allium species, and other vegetables. Sci. hortic. 2001, 89 (4): 249-256.

## Claims

1. A method of producing an antioxidant composition, comprising the steps of:

    (a) macerating tissue from plants of the genus *Allium* and incubating the tissue at a temperature less than about 30° C;

    (b) neutralizing the macerated tissue with an alkaline hydroxide or an alkali-earth hydroxide;

    (c) drying the tissue;

    (d) grinding the tissue;

    (e) extracting the tissue with a solvent; and

    (f) evaporating the solvent to recover a solid residue comprising pyruvate.

2. The method of claim 1 wherein the solvent comprises ethanol.

3. The method of claim 1 wherein the macerated tissue is incubated at a temperature less than about 25° C for about 5 minutes or longer.

4. The method of claim 3 wherein the macerated tissue is incubated at about 4° C for about 30 minutes.

5. The method of claim 1 wherein the macerated tissue is neutralized with sodium hydroxide or calcium hydroxide.

6. The method of claim 2 wherein the solvent comprises 95% ethanol (v:v)

7. The method of one of claims 1 to 6 wherein the dried tissue is defatted with hexane and residual hexane evaporated before grinding.

8. The method of one of claims 1 to 6 wherein the plant is selected from the group consisting of *Allium cepa* and *Allium sativum*.

9. The method of claim 8 wherein the plant is *Allium cepa*.

10. A pyruvate-enriched plant extract as prepared by a method of one of claims 1 to 6 or 9.

11. A plant extract derived from tissue from plants of the genus *Allium* wherein said extract is enriched in pyruvic acid by at least 100%.

12. The plant extract of claim 11 further comprising quercetin and kaempferol.

13. The plant extract of claim 11 wherein the plant is selected from the group consisting of *Allium cepa* and *Allium sativum*.

14. The extract of claim 13wherein the plant is *Allium cepa*.

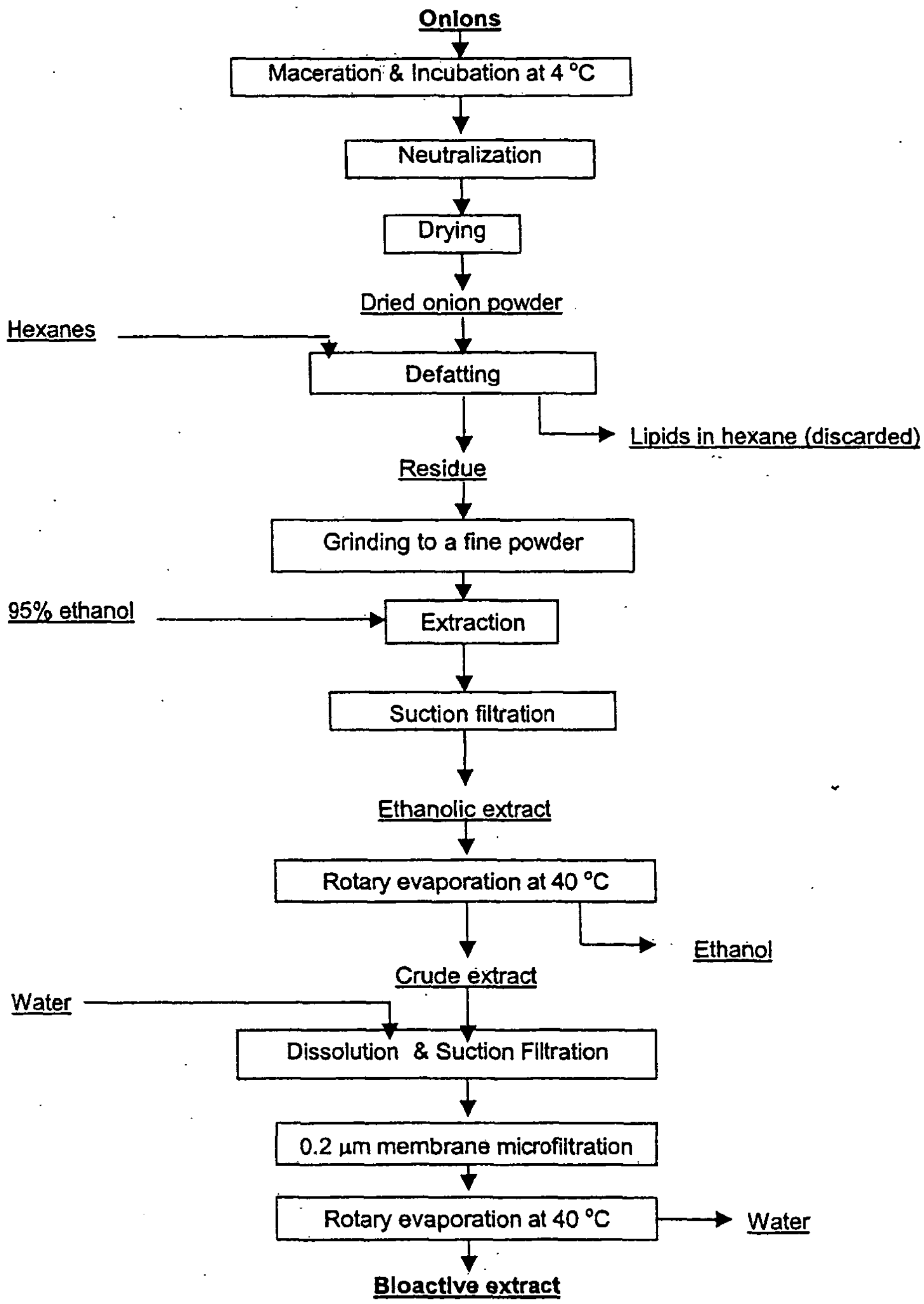


**Figure I: Onion bulb extraction scheme**

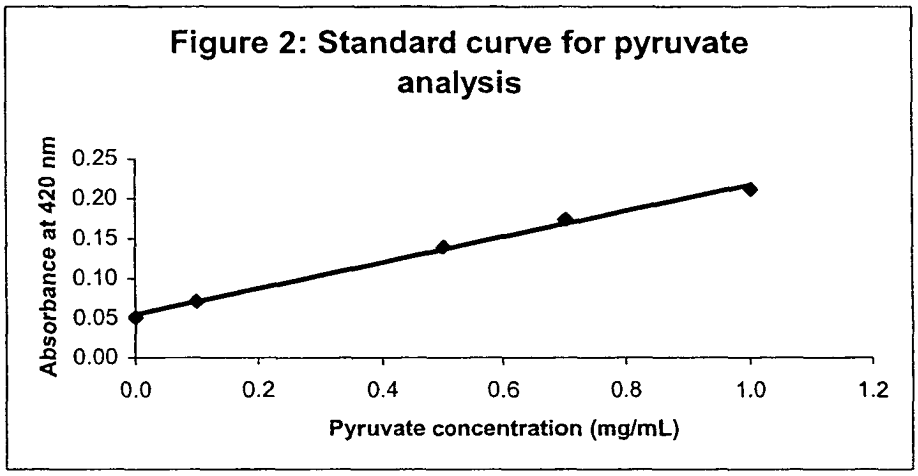
Figure 2: Standard curve for pyruvate analysis
Absorbance at 420 nm
0.25
0.20
0.15
0.10
0.05
0.00
0.0
0.2
0.4
0.6
0.8
1.0
1.2
Pyruvate concentration (mg/mL)

European Patent Office

# EUROPEAN SEARCH REPORT

Application Number
EP 04 07 7281

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br><br><br><br><br><br><br><br>Y | HANUM TIRZA ET AL: "Pyruvate and flavor development in macerated onions (Allium cepa L.) by gamma-glutamyl transpeptidase and exogenous C-S lyase"<br>FOOD CHEMISTRY,<br>vol. 54, no. 2, 1995, pages 183-188,<br>XP002299004<br>ISSN: 0308-8146<br>Results and discussion, * figures 1,2 *<br>----- | 11-14<br><br><br><br><br><br><br><br>1-10 | A63L1/00<br>A61K35/78<br>A61P39/06 |
| X<br><br>Y | US 3 725 085 A (SCHWIMMER S)<br>3 April 1973 (1973-04-03)<br>* examples 1,2 *<br>----- | 11-13<br><br>1-10 | |
| Y | DATABASE WPI<br>Section Ch, Week 200321<br>Derwent Publications Ltd., London, GB;<br>Class B04, AN 2003-218168<br>XP002299007<br>& KR 2002 086 117 A (SAMJO CELLTECH LTD)<br>18 November 2002 (2002-11-18)<br>* abstract *<br>----- | 1-10 | TECHNICAL FIELDS SEARCHED (Int.Cl.7) |
| Y | DATABASE WPI<br>Section Ch, Week 198737<br>Derwent Publications Ltd., London, GB;<br>Class D13, AN 1987-260172<br>XP002299008<br>& JP 62 179587 A (HORIUCHI KK)<br>6 August 1987 (1987-08-06)<br>* abstract *<br>-----<br>-/-- | 1-10 | A63L<br>A61K<br>A61P |

The present search report has been drawn up for all claims

| Place of search | Date of completion of the search | Examiner |
|---|---|---|
| Munich | 26 October 2004 | Perez, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 04 07 7281

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | DATABASE WPI<br>Section Ch, Week 199323<br>Derwent Publications Ltd., London, GB;<br>Class D13, AN 1993-182973<br>XP002299009<br>& BR 9 103 578 A (DE OLIVEIRA S R)<br>30 March 1993 (1993-03-30)<br>* abstract *<br>----- | 1-10 | |
| Y | DATABASE WPI<br>Section Ch, Week 198142<br>Derwent Publications Ltd., London, GB;<br>Class D13, AN 1981-76878D<br>XP002299010<br>& JP 56 113284 A (TANABE SEIYAKU CO)<br>7 September 1981 (1981-09-07)<br>* abstract *<br>----- | 1-10 | |
| D,A | BACON JAMES R ET AL: "Quantitative analysis of flavour precursors and pyruvate levels in different tissues and cultivars of onion (Allium cepa)"<br>FOOD CHEMISTRY,<br>vol. 64, no. 2, February 1999 (1999-02), pages 257-261, XP002299005<br>ISSN: 0308-8146<br>* the whole document *<br>----- | | TECHNICAL FIELDS SEARCHED (Int.Cl.7) |
| D,A | YOO KIL SUN ET AL: "Determination of background pyruvic acid concentrations in onions, Allium species, and other vegetables"<br>SCIENTIA HORTICULTURAE (AMSTERDAM),<br>vol. 89, no. 4, 30 July 2001 (2001-07-30), pages 249-256, XP002299006<br>ISSN: 0304-4238<br>* the whole document *<br>----- | | |

The present search report has been drawn up for all claims

| Place of search | Date of completion of the search | Examiner |
|---|---|---|
| Munich | 26 October 2004 | Perez, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 04 07 7281

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2004

| Patent document cited in search report | | Publication date | Patent family member(s) | Publication date |
|---|---|---|---|---|
| US 3725085 | A | 03-04-1973 | NONE | |
| KR 2002086117 | A | 18-11-2002 | NONE | |
| JP 62179587 | A | 06-08-1987 | NONE | |
| BR 9103578 | A | 30-03-1993 | NONE | |
| JP 56113284 | A | 07-09-1981 | NONE | |

EPO FORM P0459

For more details about this annex : see Official Journal of the European Patent Office, No. 12/82